# EUROPEAN PATENT SPECIFICATION

(11) **EP 0 547 666 B1**
(45) Date of publication and mention of the grant of the patent: **25.10.1995**
(21) Application number: 92203796.5
(22) Date of filing: 07.12.1992
(51) Int. Cl.: C01G 3/00, C01G 9/00, C01G 23/00, A61K 7/16

(54) **Novel antibacterial metal-ions releasing minerals**
Mineralien, die Metall-Ionen mit antibakterieller Wirkung freisetzen
Minéraux délivrant des ions métalliques antibactériens

(30) Priority: 18.12.1991 GB 9126888
(43) Date of publication of application: 23.06.1993
(73) Proprietor: UNILEVER N.V., 3000 DK Rotterdam (NL); UNILEVER PLC, London EC4P 4BQ (GB)
(72) Inventor: Creeth, A.M., Unilever Res. Port Sunlight Lab., Wirral, Merseyside L63 3JW (GB); Molyneux, H.L., Unilever Res. Port Sunlight Lab., Wirral, Merseyside L63 3JW (GB)
(74) Representative: van Gent, Jan Paulus

(56) References cited:
- EP-A- 0 368 420
- EP-A- 0 425 002

## Description

The present invention relates to novel mineral materials which are capable of releasing copper- and zinc ions, which are both effective antibacterial agents as soluble aqueous cations.

Mineral materials which are capable of releasing copper- and zinc ions have been described in European Patent Application 0,368,420 (Unilever), laid open to public inspection on 16 May 1990. The mineral materials described therein are hydrotalcite-like materials. These materials have an OH/(total metal) ratio of 2, and they can release antibacterial metal ions such as copper- and zinc ions during their slow dissolution in oral fluids.

The present invention now provides different, novel mineral materials which are also capable of releasing copper- and zinc ions and which are more effective than the above-mentioned hydrotalcite-like materials, and which have an OH/(total metal) ratio of 12/7.

The novel mineral materials according to the present invention are mixed metal basic salts with a layered crystal structure having the following general formula

CuₓZn_{y}Ti(OH)₁₂(SO_{4,}CO₃)₂.nH₂O

wherein the sum of x and y is 6, x and y being any number or fraction thereof greater than 0 and smaller than 6, and n is any number varying from 1 to 6. Preferably, x ranges from 1.5 to 4 and y from 4.5 to 2.
Preferably, the ratio of y:x varies from 1-3.

Typical examples of these novel materials are Cu₃Zn₃Ti(OH)₁₂(SO₄)₂.3H₂O and Cu_{1.6}Zn_{4.4}Ti(OH)₁₂(SO₄)₂.3H₂O.

The novel mineral materials of the present invention have a layered crystal structure and are isostructural with the mineral Spangolite, described by Ankinovich and Potok in Tr. Inst. Geol. Nauk. Kaz. SSR 15 (1966), page 145 and by Frondel, Amer. Miner. 34 (1939) page 181.

The novel mineral material consists of an agglomerated mixture of very small crystallites (with a particle size in the order of magnitude of 5 nm) together with larger platelets (sometimes as large as 0.4 µm). Depending upon their method of preparation, the novel mineral materials are usually composed of particles with a size of 20 µm or below, and usually have an average particle size, as measured in the Malvern Mastersizer, of 10 µm.

The novel mineral materials can be prepared by mixing an aqueous solution of a copper-, zinc- and titaniumsalt with an alkaline aqueous solution of an alkalimetal hydroxide, optionally with an alkalimetalcarbonate, heating the result and filtering, washing and drying the result.

The novel mineral materials release copper- and zinc ions, which are effective antibacterial cations, and therefore these novel materials can be advantageously used for delivering antibacterial properties to a variety of products, such as oral products, deodorants, antiperspirants, foot-care products, packaging materials etc.

Due to their small particle size, they are readily deposited onto the substrate and are retained thereon, thus being able to deliver the antibacterial cations quite effectively at their potential site of action.

The novel materials have been found to be particularly useful anti-plaque agents for use in oral products such as toothpastes and mouthwashes. Since they release the copper- and zinc ions under mildly acidic conditions, they are capable of releasing these ions in the oral fluids which are mildly acidic and thus combating the growth of dental plaque.

Thus, in a preferred embodiment of the present invention the novel mineral materials are used as anti-plaque agents in an oral composition.

The novel mineral material is present in such an oral composition in combination with a suitable diluent or carrier, for example a mouthwash or toothpaste. The novel mineral material is desirably present in an amount of at least about 0.01% up to about 30% by weight although greater amounts may be used. Preferred amounts are from 0.1% to 15% by weight of the oral composition.

The oral composition will contain other ingredients commonly used to formulate such products, depending on the form of the oral product. For instance, in the case of an oral composition in the form of a toothpaste the product will comprise a particulate abrasive cleaning agent, a humectant-containing liquid phase and a binder or thickener which acts to maintain the particulate solid abrasive in stable suspension in the liquid phase. A surfactant and a flavouring agent are also usual ingredients of commercially acceptable toothpastes.

Particulate solid abrasive cleaning agents commonly present in the toothpastes include silica, alumina, hydrated alumina, calcium carbonate, anhydrous dicalcium phosphate, dicalcium phosphate dihydrate, particulate hydroxyapatite and water-insoluble sodium metaphosphate. The amount of abrasive agent is usually between about 5% and 70% by weight of the toothpaste.

Humectants commonly used are glycerol and sorbitol syrup (usually comprising an approximately 70% solution). However, other humectants are known to those in the art including propylene glycol, lactitol, xylitol and hydrogenated corn syrup. The amount of humectant will generally range from about 10% of 85% by weight of the dentifrice. The remainder of the liquid phase will consist substantially of water.

Likewise numerous binding or thickening agents have been indicated for use in dentifrices, preferred ones being hydroxyethylcellulose, sodium carboxymethylcellulose and xanthan gum. Others include natural gum binders such as gum tragacanth, gum karaya and gum arabic, Irish moss, alginates and carrageenans. Silica thickening agents include the silica aerogels and various precipitated silicas. Mixtures of binders may be used. The amount of binder included in a dentifrice is generally between 0.1% and 10% by weight.

It is usual to include a surfactant in a dentifrice and again the literature discloses a wide variety of suitable materials. Surfactants which have found wide use in practice are sodium lauryl sulphate, sodium dodecylbenzene sulphonate and sodium lauroylsarcosinate.

Other anionic surfactants may be used as well as other types such as cationic, amphoteric and non-ionic surfactants. Surfactants are usually present in an amount of form 0.5% to 5% by weight of the dentifrice.

Flavours that are usually used in dentifrices are those based on oils of spearmint and peppermint. Examples of other flavouring materials used are menthol, clove, wintergreen, eucalyptus and aniseed. An amount of from 0.1% to 5% by weight is a suitable amount of flavour to incorporate in a dentifrice.

The oral composition of the invention may include a wide variety of optional ingredients. These include sweetening agent such as saccharin; an opacifying agent, such as titanium dioxide; a preservative, such as formalin; a colouring agent; or a pH controlling agent such as an acid, base or buffer, such as benzoic acid. Furthermore, they may include anti-caries agents such as sodium fluoride, stannous fluoride, monosodium fluorophosphate; anti-plaque agents such as stannous pyrophosphate, zinc citrate; antibacterial agents such as 2,4,4'-trichloro-2'-hydroxy-diphenylether, anti-calculus agents such as alkali metal pyrophosphates, anti-sensitive teeth agents and so on.

For a fuller discussion of the formulation of oral compositions reference is made to Harry's Cosmeticology, Seventh Edition, 1982, Edited by J B Wilkinson and R J More, pages 609 to 617.

The invention also provides a method of treating the oral cavity with the above novel mineral materials. The treatment may comprise rinsing with a suspension of the novel mineral material in water or with a flavoured mouthwash product containing the novel mineral material or by brushing the teeth with a dental product comprising the novel mineral material.

### EXAMPLE 1

### 1. Synthesis of sample 1

Solution A was prepared by mixing 20.7 g of ZnSO₄.7H₂O and 18.0 g of CuSO₄.5H₂O with 37 g of a 15% Ti(SO₄)₂ solution (BDH Technical) and then diluting with 300 cm³ of water. Solution B was made up by dissolving 57.2 g of Na₂CO₃ and 22.0 g of NaOH in 400 cm³ of water. A large beaker containing 200 cm³ of water was set up with a Heidolph stirrer. The stirring rate was set to 500 rpm, then solutions A and B were added simultaneously using peristaltic pumps, with the rates of addition adjusted so that the pH of the mixture was maintained at pH 7. Additions were continued until all of solution A was used up. The result was placed in an oven set at 95°C for 24 hours and the product filtered, washed with water and dried overnight at 95°C.

### 2. Analysis

a) XRD : Intensities of the peaks found are given below.

| d/Å | Iᵣₑₗ | d/Å | Iᵣₑₗ | d/Å | Iᵣₑₗ |
|---|---|---|---|---|---|
| 7.32 | 100 | 2.70 | 52 | 1.80 | 19 |
| 5.11 | 5 | 2.53 | 51 | 1.74 | 9 |
| 4.12 | 16 | 2.38 | 9 | 1.56 | 19 |
| 3.58 | 39 | 2.16 | 18 | 1.53 | 14 |
| 3.12 | 26 | 1.98 | 8 | 1.49 | 9 |

b) Composition : AA analysis.

| | |
|---|---|
| Zn | 21.9% |
| Cu | 18.5% |
| Ti | 4.6% |
| Na | 2.8% |
| SO₄ | 2.7% |

Total volatile material at 900°C 28.3%
The composition corresponds to the formula

Zn_{3.5}Cu₃Ti(OH)₁₂(SO₄)₂.3H₂O

with an impurity of Na₂SO₄ and a small one of Na₂CO₃. On the basis of the XRD and AA analysis, the material was found to be in good agreement with the structure

Zn₃Cu₃Ti(OH)₁₂(SO₄)₂.3H₂O

.

### 3. Metal ion release by acid

A suspension of 0.5 g of the above sample was prepared in 50 cm³ of water. The mixture was agitated with a Heidolph stirrer and pH-statted at a set pH with 1 M HNO₃ for 300 seconds. An aliquot (0.5 cm³) was removed, diluted to 10 cm³ and centrifuged 200 rpm for 10 minutes. The supernatant was analysed for Cu²⁺ and Zn²⁺ by atomic absorption spectroscopy. The results are given below.

| Concentration of metal ions in original suspension/ppm. | | |
|---|---|---|
| pH | Cu²⁺ | Zn²⁺ |
| 7 | 30 | 160 |
| 6.5 | 40 | 280 |
| 6 | 130 | 530 |
| 5.5 | 140 | 680 |
| 5 | 430 | 1080 |
| 4.5 | 985 | 1615 |
| 4 | 1410 | 2090 |

### 4. Anti-plaque effect

A suspension of the above sample (20 µl per disc) was carefully pipetted onto acid-washed hydroxyapatite discs (Synamel, 8 mm diameter). Discs were oven-dried at 37°C to produce a film of the sample on the upper surface of the disc. The effect of increasing amounts of this sample on plaque growth was determined, using an **in vitro** plaque test. Discs coated with this sample were inoculated with **Streptococcus sanguis** 209 and then incubated at 38°C for 18 hours under intermittent exposure to sterile Brain Heart Infusion broth (+ 1% sucrose) in an **in vitro** plaque growth model. For the first two dose response experiments (Experiments 1 and 2), the plaque-covered discs were transferred into a vial containing BDH Analar water (2 ml). The vial was sonicated (20 seconds) to disperse the plaque, and then the plaque growth was determined by viability counting of bacteria spiral plated on blood agar. For the final dose response experiment 3, only plaque growth on the upper surface of the disc was assessed (i.e. the sample-coated region). This was done by swabbing plaque from the top of the disc, using a calcium alginate swab, followed by dispersing the plaque in Analar water (2 ml) and counting as before.

In all three experiments the major antiplaque dose response was seen between 0 and 1 µg Cu (supernatant). One hundred percent inhibition of plaque growth was achieved at 0.8 µg Cu (experiment 3).

The results are given below.

| Experiments 1 and 2 | | |
|---|---|---|
| inhibition inhibition Amount of CU delivered (µg) | % plaque growth | |
| | Experiment 1 | Experiment 2 |
| 0 | 0 | 0 |
| 0.2 | 42 | 22 |
| 0.5 | 53 | 78 |
| 1 | 74 | 100 |
| 2 | 73 | 90 |
| 3 | 76 | - |
| 4 | - | 80 |
| 4.6 | - | 100 |
| 5.8 | 100 | - |

| Experiment 3 | |
|---|---|
| Amount of Cu delivered (µg) | % plaque growth inhibition |
| 0 | - |
| 0.2 | 50 |
| 0.4 | 68 |
| 0.6 | 95 |
| 0.8 | 100 |

### EXAMPLE 2

Solutions of 8.99 g of CuSO₄.5H₂O in 100 ml of water, 31.05 g of ZnSO₄.7H₂O in 100 ml water and 37 g of a 15% solution of Ti(SO₄)₂ in 100 ml of water were mixed together. The mixture was stirred at 2000 rpm. with an overhead stirrer and 155 ml of a 2M NaOH solution added via a dropping funnel. The result was placed in an oven at 95°C for 40 hours and treated as for the previous method.

| XRD Pattern | | | | | |
|---|---|---|---|---|---|
| d/Å | Iᵣₑₗ | d/Å | Iᵣₑₗ | d/Å | Iᵣₑₗ |
| 7.28 | 100 | 2.71 | 61 | 1.80 | 18 |
| 5.11 | 4 | 2.54 | 63 | 1.74 | 8 |
| 4.14 | 13 | 2.40 | 6 | 1.56 | 22 |
| 3.59 | 33 | 2.16 | 17 | 1.53 | 10 |
| 3.21 | 24 | 1.99 | 6 | 1.49 | 11 |

| Composition (by AA analysis) | |
|---|---|
| Zn | 34.9% |
| Cu | 12.7% |
| Ti | 5.8% |
| SO₄ | 8.3% |
| Na | 0.3% |
| Total volatile material | 28.0% |

On the basis of the XRD and AA analysis, the material was found to correspond to the formula

Zn_{4.4}Cu_{1.6}Ti(OH)₁₂(SO₄)₂.3H₂O

.

## Claims

1. Novel compounds of the general formula
CuₓZn_{y}Ti(OH)₁₂(SO_{4,}CO₃)₂.nH₂O
in which x and y are any number or fraction thereof, greater than 0 and smaller than 6, the sum of x and y being 6, and n is any number between 1 and 6.

2. The compounds of claim 1, in which x ranges from 1.5 to 4 and y ranges from 4.5 to 2.

3. The compounds of claim 1, in which the ratio of y:x ranges from 1 to 3.

4. An antibacterial composition comprising an effective amount of a compound according to claims 1-3.

5. An oral care composition comprising from 0.01-30% by weight of a compound according to claims 1-3.

6. Use of a compound according to claims 1-3 as an anti-plaque agent in oral care products.

## Patentansprüche

1. Neue Verbindungen der allgemeinen Formel
CuₓZn_{y}Ti(OH)₁₂(SO₄,CO₃)₂·H₂O ,
worin x und y eine beliebige Zahl oder einen Bruchteil davon darstellen, größer als 0 und kleiner als 6, wobei die Summe von x und y 6 ist und n eine beliebige Zahl zwischen 1 und 6 ist.

2. Verbindungen nach Anspruch 1, wobei x im Bereich von 1,5 bis 4 liegt und y im Bereich von 4,5 bis 2 liegt.

3. Verbindungen nach Anspruch 1, wobei das Verhältnis von y:x im Bereich von 1 bis 3 liegt.

4. Antibakterielles Mittel, umfassend eine wirksame Menge einer Verbindung nach Ansprüchen 1 bis 3.

5. Mundpflegemittel, umfassend 0,01 bis 30 Gew.-% einer Verbindung nach Ansprüchen 1 bis 3.

6. Verwendung einer Verbindung nach Ansprüchen 1 bis 3 als Antiplaquemittel in Mundpflegeprodukten.

## Revendications

1. Nouveaux composés répondant à la formule générale
CuₓZn_{y}Ti(OH)₁₂(SO₄, CO₃)₂, nH₂O
dans laquelle x et y sont des nombres ou des fractions de nombres supérieurs à 0 et inférieurs à 6, la somme de x et de y étant égale à 6, et n étant un nombre pouvant prendre toutes les valeurs de 1 à 6.

2. Les composés de la revendication 1, dans lesquels x est compris entre 1,5 et 4 et y est compris entre 4,5 et 2.

3. Les composés de la revendication 1, dans lesquels le rapport de y:x est compris entre 1 et 3.

4. Une composition antibactérienne contenant une quantité efficace d'un composé selon les revendications 1 à 3.

5. Une composition pour les soins buccaux contenant de 0,01-30% en poids d'un composé selon les revendications 1 à 3.

6. L'utilisation d'un composé selon les revendications 1-3 comme agent anti-plaque dans des produits pour soins buccaux.
